# EUROPÄISCHE PATENTSCHRIFT

(11) **EP 0 422 366 B1**
(45) Veröffentlichungstag und Bekanntmachung des Hinweises auf die Patenterteilung: **22.01.1997**
(21) Anmeldenummer: 90116212.3
(22) Anmeldetag: 24.08.1990
(51) Int. Cl.: C07D 207/267, C07D 207/38

(54) **Verfahren zur Herstellung N-substituierter Pyrrolidin-2-one**
Process for the preparation of N-substituted pyrolidin-2-ones
Procédé pour la préparation de pyrolidin-2-ones N-substitués

(30) Priorität: 01.09.1989 DE 3928982; 01.09.1989 DE 3928981
(43) Veröffentlichungstag der Anmeldung: 17.04.1991
(73) Patentinhaber: BASF Aktiengesellschaft, 67063 Ludwigshafen (DE)
(72) Erfinder: Schuster, Ludwig, Dr., D-6703 Limburgerhof (DE); Koehler, Ulrich, Dr., D-6900 Heidelberg (DE)

(56) Entgegenhaltungen:
- DE-B- 1 014 113
- FR-A- 1 171 323
- US-A- 4 420 620
- CHEMICAL ABSTRACTS, vol. 45, no. 18, 25. September 1951, Columbus, Ohio, US; abstract no. 7953D, M. PROTIVA ET AL: 'Derivatives of beta- cyanopropionic acid'
- ULLMANN'S ENCYCLOPEDIA OF INDUSTRIAL CHEMISTRY, 5te Auflage, Band A1' 1985 , VCH VERLAGSGESELLSCHAFT MBH , WEINHEIM, DE; Seite 165

## Beschreibung

Die vorliegende Erfindung betrifft ein Verfahren zur Herstellung N-substituierter Pyrrolidin-2-one aus N,N'-disubstituierten 5-Imino-pyrrolidin-2-onen an Hydrierkatalysatoren bei erhöhten Drücken und Temperaturen.

N-substituierte Pyrrolidin-2-one, insbesondere N-Methylpyrrolidon (NMP) der Formel III 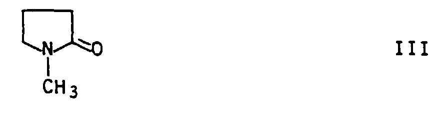 können im industriellen Maßstab durch Umsetzung von Butyrolacton mit den jeweiligen primären Aminen, insbesondere Methylamin, gewonnen werden. Dieses Verfahren hat sich insbesondere zur Herstellung von NMP bewährt. Butyrolacton wird durch katalytische Dehydrierung aus 1,4-Butandiol hergestellt, dessen Herstellung z.B. auf dem Rohstoff Acetylen beruht (vgl. dazu: Weissermel, Arpe: Industrielle organische Chemie, Seite 105 bis 109, VCH-Verlagsgesellschaft, Weinheim 1988).

Gemäß DE-B 1 614 113 wird bei der Hydrierung von β-Cyanpropionsäurealkylestern in Gegenwart von Ammoniak direkt das unsubstituierte 2-Pyrrolidon erhalten. Nach US-A 4 420 620 werden N-substituierte 2-Pyrrolidone durch die Umsetzung von 4-Oxobuttersäuremethylester mit Aminen in Gegenwart katalytischer Mengen Wasserstoff an einem Hydrierkatalysator erzeugt.

Da die Wettbewerbsfähigkeit von Verfahren zur Herstellung chemischer Großprodukte sehr stark von den Preisen für die erforderlichen Rohstoffe abhängig ist, diese aber wiederum aufgrund der Angebotslage auf dem Weltmarkt und infolge der Verfügbarkeit von Rohstoffquellen und chemischen Anlagen zu deren kostengünstigen Herstellung zeitlich als auch örtlich stark variieren können, bestand die Aufgabe, ein wirtschaftliches Verfahren zur Herstellung von N-substituierten Pyrrolidin-2-onen zu finden, das auf einer zu Acetylen alternativen Rohstoffbasis beruht.

Demgemäß wurde ein neues und verbessertes Verfahren zur Herstellung N-substituierter Pyrrolidin-2-one der allgemeinen Formel I 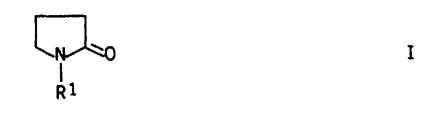 in der R¹ für eine C₁- bis C₂₀-Alkyl-, eine C₆- bis C₁₀-Aryl- oder eine C₇- bis C₁₂-Aralkylgruppe steht, gefunden, welches dadurch gekennzeichnet ist, daß man 5-Imino-pyrrolidin-2-one der allgemeinen Formel II 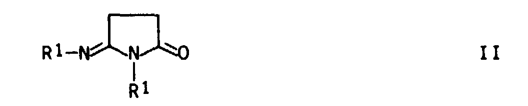 in der R¹ gleich oder verschieden sein können und eine C₁- bis C₂₀-Alkyl-, eine C₆- bis C₁₀-Aryl-oder eine C₇- bis C₁₂-Aralkylgruppe bedeuten, bei erhöhtem Druck und bei erhöhter Temperatur in Gegenwart eines Hydrierkatalysators hydrogenolytisch zu den Pyrrolidin-2-onen I spaltet. Im speziellen lassen sich die 5-Imino-pyrrolidone der Formel II durch Umsetzung von β-cyanopropionestern der allgemeinen Formel III 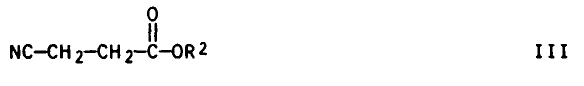 in der R² eine C₁- bis C₂₀-Alkyl-, eine C₇- bis C₁₂-Aralkyl- oder eine C₆- bis C₁₀-Arylgruppe bedeutet, mit einem Amin der allgemeinen Formel IV

R¹-NH₂ IV

in der R¹ die oben genannte Bedeutung besitzt, erhalten.

Herstellung der 5-Imino-pyrrolidin-2-one der allgemeinen Formel II:

Der als Ausgangsverbindungen verwendeten β-cyanopropionsäureester III sind in praktisch quantitativer Ausbeute durch Addition von Blausäure an die entsprechenden Acrylsäureester erhältlich. Dadurch erniedrigt sich die Zahl der Reaktionsschritte zur Herstellung der Verbindung III, wie aus dem folgenden Schema ersichtlich, auf 2 Stufen.

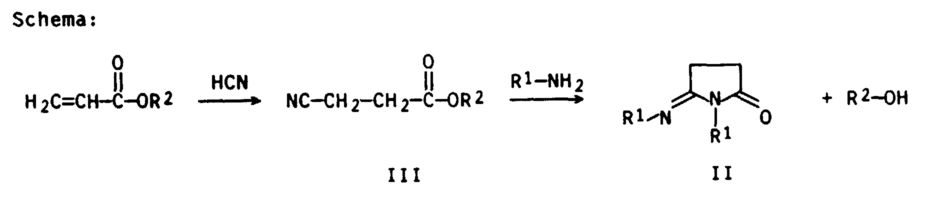

Die Art der Esterkomponente R² der β-Cyanopropionsäureester III spielt für das Gelingen des erfindungsgemäßen Verfahrens nur eine untergeordnete Rolle, weshalb beispielsweise sowohl C₁- bis C₂₀-Alkyl-, C₇- bis C₁₂-Aralkyl- als auch C₆- bis C₁₀-Arylgruppen als Esterkomponente R² Verwendung finden können. Es versteht sich von selbst, daß die Reste R² nicht so raumerfüllend sein sollten, daß sie eine sterische Hinderung der Reaktion verursachen. Aufgrund ihrer leichten Verfügbarkeit und Preiswürdigkeit werden jedoch vorzugsweise β-cyanopropionester III verwendet, in denen R² für ein C₁- bis C₄-Alkylgruppe steht. Selbstverständlich können die Reste R2 auch mit unter den Reaktionsbedingungen inerten Substituenten substituiert sein.

Die Art der Reste R¹ in den Verbindungen II ist natürlich von der Natur der zur Cyclisierung der β-Cyanopropionsäureester III verwendeten Amine IV - R¹-NH₂- abhängig. Zur Cyclisierung der Ester III eignen sich praktisch alle primären Amine R¹-NH₂, d.h. Amine, in denen R¹ für einen aliphatischen Rest mit 1 bis 20, einen aromatischen Rest mit 6 bis 10 oder einen araliphatischen Rest mit 7 bis 12 Kohlenstoffatomen steht, vorausgesetzt die Reste R¹ sind sterisch nicht so anspruchsvoll, daß die Reaktion sterisch gehindert wird. Dementsprechend können Amine IV, in denen der Rest R¹ für eine C₁- bis C₂₀-Alkyl-, C₆- bis C₁₀-Aryl- oder eine C₇- bis C₁₂-Aralkylgruppe, verwendet werden. Des weiteren können Amine IV verwendet werden, welche in ihrem aliphatischen, cycloaliphatischen, aromatischen oder araliphatischen Rest Heteroatome wie Sauerstoff, Stickstoff oder Schwefel enthalten. Bevorzugt werden Amine IV eingesetzt, in denen R¹ eine C₁- bis C₄-Alkylgruppe und besonders bevorzugt die Methylgruppe ist. Es versteht sich von selbst, daß sich die Anwendung von Aminen IV, in denen R¹ mit unter den Reaktionsbedingungen inerten Substituenten substituiert ist, auf den Erfolg des Verfahrens nicht nachteilig auswirkt. Als Beispiele für solche Substituenten seien Ethergruppen oder Acetalgruppierungen genannt.

Üblicherweise wird der β-Cyanpropionsäureester III nur mit einer Aminspezies R¹-NH₂ umgesetzt. Es ist natürlich auch möglich, diesen Ester mit einer Mischung von Aminen R¹-NH₂, mit unterschiedlichen Resten R¹, umzusetzen. Dabei entstehen Produktgemische, welche Verbindungen II enthalten, in denen die Reste R¹ verschieden sind.

Die Amine IV können im erfindungsgemäßen Verfahren bezüglich des β-Cyanopropionsäureester III sowohl in stöchiometrischer Menge, d.h. in einer Menge von 2 mol Amin IV/mol Ester III, als auch im Überschuß bezüglich III eingesetzt werden. Zweckmäßigerweise wird ein Überschuß an Aminen IV gearbeitet, wobei im allgemeinen 2,2 bis 10, vorzugsweise 2,5 bis 4 mol Amin IV/mol Ester III eingesetzt werden.

Die Umsetzung der Amine IV mit dem β-Cyanopropionsäureester III kann in einem unter den Reaktionsbedingungen inerten Lösungsmittel, wie Tetrahydrofuran oder Dioxan, ausgeführt werden. Vorzugsweise wird die Umsetzung jedoch in Abwesenheit eines zusätzlichen Lösungsmittels genommen, wobei jedoch gegebenenfalls das im Überschuß zugegebene Amin III oder aber auch zugesetztes Verfahrensprodukt I als Lösungsmittel dienen kann. Lösungsmittel werden insbesondere dann dem Reaktionsansatz zugesetzt, wenn Ausgangsmaterialien und/oder Verfahrensprodukt I Feststoffe sind.

Die Umsetzung kann sowohl bei Atmosphärendruck als auch unter erhöhtem Druck durchgeführt werden. Unter erhöhtem Druck, insbesondere autogen erzeugtem Druck, wird zweckmäßigerweise dann gearbeitet, wenn einer der Reaktanten, im allgemeinen das Amin IV, unter den Reaktionsbedingungen siedet. Ebenso ist es möglich einen unter den Reaktionsbedingungen gasförmigen Reaktanten, beispielsweise Methylamin, gasförmig bei Atmosphärendruck in das flüssige Reaktionsgemisch einzuleiten.

Die beiden Reaktanten β-Cyanpropionsäureester III und das Amin IV können bereits bei Raumtemperatur miteinander reagieren. Zur Beschleunigung und Vervollständigung der Umsetzung wird jedoch im allgemeinen bei höheren Temperaturen gearbeitet. Zweckmäßigerweise wird die Umsetzung bei Temperaturen zwischen 20 und 250°C, vorzugsweise bei Temperaturen von 30 bis 150°C insbesondere bei 90 bis 130°C durchgeführt. Wie bereits erwähnt, wird die Umsetzung zweckmäßigerweise unter dem von den Reaktanten bei diesen Temperaturen erzeugten Eigendruck ausgeführt. Eine weitere exogene Erhöhung des Reaktionsdruckes ist möglich, erweist sich aber im allgemeinen als nicht erforderlich.

Die 5-Imino-pyrrolidin-2-one II können aus dem Reaktionsaustrag auf herkömmliche Weise isoliert werden, beispielsweise durch Destillation oder Kristallisation. Dabei werden die Verbindungen II in hoher Reinheit erhalten. Der im Zuge der Umsetzung freigesetzte Alkohol R²OH kann durch Destillation zurückgewonnen und wiederverwendet werden.

Das erfindungsgemäße Verfahren kann je nach Bedarf diskontinuierlich oder kontinuierlich ausgeführt werden. Dabei können die für diese Betriebsweisen üblicherweise verwendeten Reaktoren eingesetzt werden - beispielsweise Rühr- oder Blasenreaktoren im diskontinuierlichen, Rohrreaktoren im kontinuierlichen Betrieb.

Die hydrogenolytische Spaltung der 5-Imino-pyrrolidin-2-one der allgemeinen Formel II verläuft formal nach der folgenden Reaktionsgleichung:

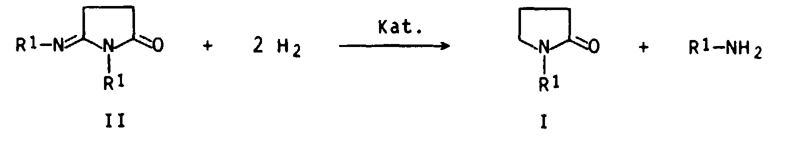

Nach dem erfindungsgemäβen Verfahren lassen sich auf vorteilhafte Weise N-substituierte Pyrrolidin-2-one der Formel I herstellen, in denen R¹ eine C₁- bis C₂₀-Alkyl-, C₆- bis C₁₀-Aryl- oder C₇- bis C₁₂-Aralkylgruppe ist. Besonders vorteilhaft gestaltet sich nach dem erfindungsgemäβen Verfahren die Herstellung von N-substituierten Pyrrolidin-2-onen, in denen R¹ eine C₁- bis C₄-Alkylgruppe und insbesondere die Methylgruppe ist.

Als Ausgangsmaterialien zur Herstellung der N-substituierten Pyrrolidin-2-one I dienen die 5-Imino-pyrrolidin-2-one II, in denen das Imido-Stickstoff-Atom den entsprechenden Rest R¹ trägt und in denen das Imino-Stickstoff-Atom auch unsubstituiert sein kann oder eine C₁- bis C₂₀-Alkyl-, eine C₆- bis C₁₀-Aryl- oder eine C₇- bis C₁₂-Aralkylgruppe tragen kann. R¹ in II kann gleich oder verschieden sein. Vorzugsweise werden Verbindungen II eingesetzt, in denen die Reste R¹ gleich sind und eine C₁- bis C₄-Alkylgruppe, insbesondere die Methylgruppe bedeuten.

Zur hydrogenolytischen Abspaltung der Iminogruppierung der 5-Iminopyrrolidin-2-one II eignen sich im Prinzip alle Hydrierkatalysatoren, d.h. unter der Vielzahl der getesteten Hydrierkatalysatoren konnte bislang keiner gefunden werden, mit dem sich die erfindungsgemäße Umsetzung nicht durchführen ließ. Als geeignete Katalysatoren seien beispielhaft die folgenden Hydrierkatalysatoren genannt: Platinmetalle wie Platin, Palladium, Rhodium und Ruthenium sowie deren Oxide und Oxidhydrate, Raney-Nickel, Raney-Kobalt, Raney-Kupfer, Eisen oder Eisen(III)oxid. Diese Hydrierkatalysatoren können als solche in reiner Form, vorzugsweise in fein verteilter Form mit großer Oberfläche, eingesetzt werden. Es kann aber auch vorteilhaft sein, diese Katalysatoren auf einem inerten Trägermaterial abgeschieden, also als Trägerkatalysatoren, einzusetzen. Dazu können alle für Hydrierkatalysatoren bewährten Trägermaterialien wie Aluminiumoxide, Zirkondioxid, Bariumsulfat oder Aktivkohle benutzt werden.

Selbstverständlich ist es auch möglich Mischkatalysatoren als Hydrierkatalysatoren im erfindungsgemäßen Verfahren zu verwenden. Beispielhaft für derartige Katalysatoren seien die Hydrierkatalysatoren der DE-PS 1 235 879 oder die Klasse der Hydrier-/Dehydrier-Katalysatoren auf der Basis von Chromiten genannt (siehe z.B. US-A 2 492 614; US-A 1 977 750; J. Am. Chem. Soc. 62, 2874 bis 2876, (1940)).

Die Hydrierung wird unter erhöhtem Druck und bei erhöhter Temperatur vorgenommen. Im allgemeinen wird die erfindungsgemäße Umsetzung bei Temperaturen von 150 bis 250, vorzugsweise von 170 bis 220 und insbesondere bei Temperaturen von 180 bis 200°C sowie bei Drucken von zweckmäßigerweise 100 bis 400, vorteilhaft 200 bis 350 und besonders bevorzugt bei 250 bis 320 bar durchgeführt.

Die Hydrierung kann in An- oder Abwesenheit von unter den Reaktionsbedingungen inerten Lösungsmitteln wie Tetrahydrofuran, Dioxan, Dimethoxyethan, Kohlenwasserstoffen oder aliphatischen Alkoholen erfolgen. Der Zusatz eines Lösungsmittels zum Reaktionsansatz kann sich insbesondere dann vorteilhaft auswirken, wenn es sich bei dem umzusetzenden Ausgangsmaterial und/oder dem herzustellenden Produkt um bei Raumtemperatur feste Stoffe handelt.

Die erfindungsgemäße Umsetzung kann je nach Bedarf chargenweise in Rührautoklaven mit suspendierten Katalysatoren oder kontinuierlich in Festbettreaktoren, wahlweise in der Sumpf- oder Rieselfahrweise, durchgeführt werden. Zur Isolierung der Pyrrolidin-2-one I wird im allgemeinen so vorgegangen, daß man den Reaktionsaustrag abkühlt und entspannt, erforderlichenfalls vom Katalysator abtrennt und das Produkt durch Destillation oder Kristallisation reinigt.

Nach dem erfindungsgemäßen Verfahren lassen sich N-substituierte Pyrrolidin-2-one I wirtschaftlich konkurrenzfähig und in hoher Reinheit aus einer alternativen Rohstoffquelle gewinnen.

Die nach dem erfindungsgemäßen Verfahren erhältlichen N-substituierten Pyrrolidin-2-one I finden beispielsweise als Lösungs- und Extraktionsmittel breite Verwendung.

### Beispiele

### Beispiel 1

In einem Autoklaven wurden bei Raumtemperatur 350 g β-Cyanopropionsäuremethylester vorgelegt und 350 g gasförmiges Methylamin in den Autoklaven eingepreßt. Nach dem Abklingen der ersten Reaktion wurde noch 3 Stunden auf 130°C erhitzt, wobei sich im Autoklaven ein Druck von 20 bar einstellte. Der Reaktionsaustrag wurde unter vermindertem Druck fraktionierend destilliert. 1-Methyl-5-methylimino-2-pyrrolidinon wurde in einer Ausbeute von 98 %, bezogen auf den Ester II, und in einer Reinheit von 99,2 % erhalten.

### Beispiel 2

In einem Autoklaven von 300 ml Inhalt wurden folgende Stoffe vorgelegt:
30 g 5-N-Methylimino-N-methyl-2-pyrrolidon
130 g Tetrahydrofuran
2 g Katalysator (Palladium auf Aluminiumoxidträger; Palladiumgehalt: 5 Gew.-%)

Auf diesen Reaktionsansatz wurden bei Raumtemperatur 100 bar Wasserstoff aufgepreßt und der Autoklav unter Rühren auf 180°C hochgeheizt. Nach dem Erreichen dieser Reaktionstemperatur wurde der Druck auf 300 bar durch Aufpressen von Wasserstoff erhöht. Nach 5 Stunden Reaktionsdauer ließ man abkühlen, entspannte und arbeitete nach dem Abfiltrieren des Katalysators durch Destillation auf. Es wurden 19 g N-Methylpyrrolidon erhalten (Ausbeute: 81 %).

### Beispiel 3

Beispiel 2 wurde mit folgenden Einsatzstoffen wiederholt:
80 g 5-N-Methylimino-N-methyl-2-pyrrolidon
80 g Tetrahydrofuran
5 g Raney-Kupfer

Es wurden 38 g NMP erhalten (Ausbeute: 61,5 %).

### Beispiel 4

Beispiel 2 wurde mit folgenden Einsatzstoffen wiederholt:
80 g 5-N-Methylimino-N-methyl-2-pyrrolidon
80 g Tetrahydrofuran
5 g Katalysator gemäß DE-PS 1 235 879
   (Zusammensetzung: 20 Gew.-% Kobalt, 55 Gew.-% Kupfer, 25 Gew.-% Mangan)

Es wurden 41 g NMP erhalten (Ausbeute: 65 %).

### Beispiel 5

Beispiel 2 wurde mit folgenden Einsatzstoffen wiederholt:
80 g 5-N-Methylimino-N-methyl-2-pyrrolidon
80 g Tetrahydrofuran
5 g Raney-Nickel

Es wurden 38 g NMP erhalten (Ausbeute: 61,5 %).

## Patentansprüche

1. Verfahren zur Herstellung N-substituierter Pyrrolidin-2-one der allgemeinen Formel I 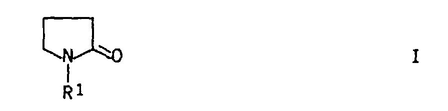 in der R¹ für eine C₁- bis C₂₀-Alkyl-, eine C₆- bis C₁₀-Aryl- oder eine C₇- bis C₁₂-Aralkylgruppe steht, dadurch gekennzeichnet, daß man 5-Imino-pyrrolidin-2-one der allgemeinen Formel II 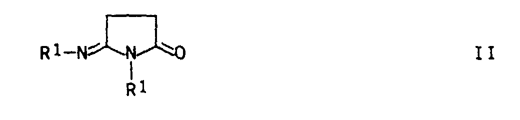 in der R¹ gleich oder verschieden sein können und eine C₁- bis C₂₀-Alkyl-, eine C₆- bis C₁₀-Aryl-oder eine C₇- bis C₁₂-Aralkylgruppe bedeuten, bei erhöhtem Druck und bei erhöhter Temperatur in Gegenwart eines Hydrierkatalysators hydrogenolytisch zu den Pyrrolidin-2-onen I spaltet.

2. Verfahren zur Herstellung N-substituierter Pyrrolidin-2-one der allgemeinen Formel I 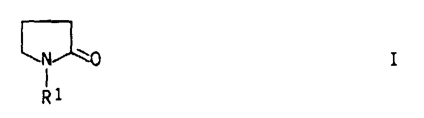 in der R¹ für eine C₁- bis C₂₀-Alkyl-, eine C₆- bis C₁₀-Aryl- oder eine C₇- bis C₁₂-Aralkylgruppe steht, dadurch gekennzeichnet, daß man β-Cyanopropionester der allgemeinen Formel III 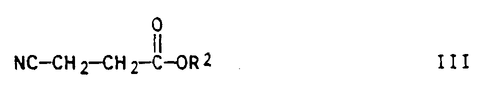 in der R² eine C₁- bis C₂₀-Alkyl-, eine C₇- bis C₁₂-Aralkyl- oder eine C₆- bis C₁₀-Arylgruppe bedeutet, mit einem Amin der allgemeinen Formel IV
R¹-NH₂ IV
in der R¹ die oben genannte Bedeutung besitzt, umsetzt und die gebildeten 5-Imino-pyrrolidin-2-one der allgemeinen Formel II 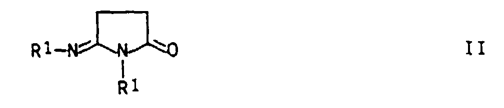 in der R¹ gleich oder verschieden sein können und eine C₁- bis C₂₀-Alkyl-, eine C₆- bis C₁₀-Aryl- oder eine C₇- bis C₁₂-Aralkylgruppe bedeuten, nach deren Isolierung bei erhöhtem Druck und bei erhöhter Temperatur in Gegenwart eines Hydrierkatalysators hydrogenolytisch zu den Pyrrolidin-2-onen I spaltet.

## Claims

1. A process for the preparation of an N-substituted pyrrolidin-2-one of the general formula I 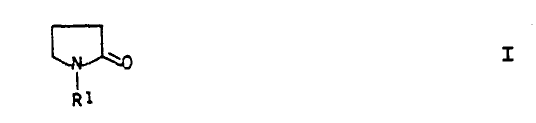 where R¹ is C₁- to C₂₀-alkyl, C₆- to C₁₀-aryl or C₇- to C₁₂-aralkyl, which comprises hydrogenolytically cleaving a 5-iminopyrrolidin-2-one of the general formula II 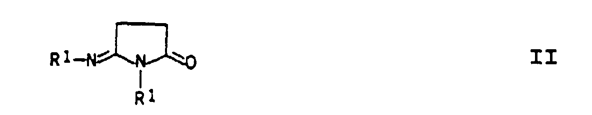 where the R¹ radicals may be identical or different C₁- to C₂₀-alkyl, C₆- to C₁₀-aryl or C₇- to C₁₂-aralkyl, at superatmospheric pressure and elevated temperature in the presence of a hydrogenation catalyst to give a pyrrolidin-2-one I.

2. A process for the preparation of an N-substituted pyrrolidin-2-one of the general formula I 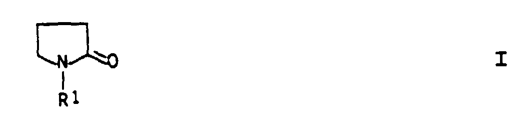 where R¹ is C₁- to C₂₀-alkyl, C₆- to C₁₀-aryl or C₇- to C₁₂-aralkyl, which comprises reacting a β-cyanopropionic ester of the general formula III 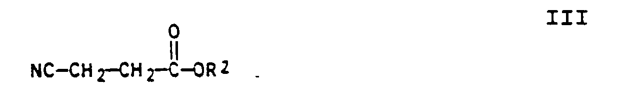 where R² is C₁- to C₂₀-alkyl, C₇- to C₁₂-aralkyl or C₆- to C₁₀-aryl, with an amine of the general formula IV
R¹-NH₂ IV
where R¹ is as defined above, and, after their isolation, hydrogenolytically cleaving the resultant 5-iminopyrrolidin-2-one of the general formula II 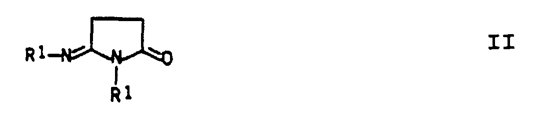 where the R¹ radicals may be identical or different C₁- to C₂₀-alkyl, C₆- to C₁₀-aryl or C₇- to C₁₂-aralkyl, at superatmospheric pressure and at elevated temperature in the presence of a hydrogenation catalyst to give a pyrrolidin-2-one I.

## Revendications

1. Procédé de préparation de pyrrolidine-2-ones N-substituées de la formule générale I 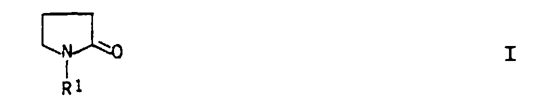 dans laquelle R¹ représente un radical alkyle en C₁ à C₂₀, un radical aryle en C₆ à C₁₀, ou un radical aralkyle en C₇ à C₁₂, caractérisé en ce que l'on scinde par hydrogénolyse des 5-iminopyrrolidine-2-ones de la formule générale II 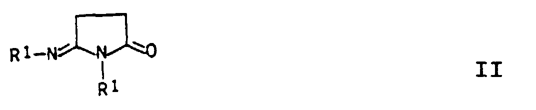 dans laquelle les symboles R¹ peuvent avoir des significations identiques ou différentes et représenter chacun un radical alkyle en C₁ à C₂₀, un radical aryle en C₆ à C₁₀, ou un radical aralkyle en C₇ à C₁₂, à température élevée et sous pression élevée, en présence d'un catalyseur d'hydrogénation, de manière à obtenir les pyrrolidine-2-ones I.

2. Procédé de préparation de pyrrolidine-2-ones N-substituées de la formule générale I 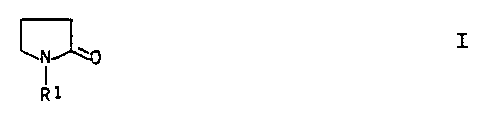 dans laquelle R¹ représente un radical alkyle en C₁ à C₂₀, un radical aryle en C₆ à C₁₀, ou un radical aralkyle en C₇ à C₁₂, caractérisé en ce que l'on fait réagir des esters de l'acide β-cyanopropionique de la formule générale III 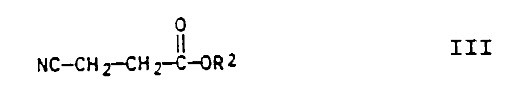 dans laquelle R² représente un radical alkyle en C₁ à C₂₀, un radical aralkyle en C₇ à C₁₂, ou un radical aryle en C₆ à C₁₀, avec une amine de la formule générale IV
R¹-NH₂ IV
dans laquelle R¹ possède les significations qui lui ont été précédemment attribuées et on scinde par hydrogénolyse les 5-iminopyrrolidine-2-ones formées de la formule générale II 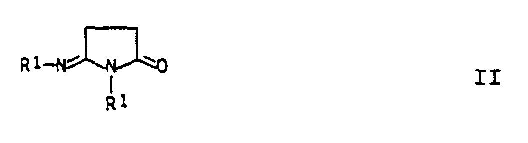 dans laquelle les symboles R¹ peuvent avoir des significations identiques ou différentes et représentent chacun un radical alkyle en C₁ à C₂₀, un radical aryle en C₆ à C₁₀, ou un radical aralkyle en C₇ à C₁₂, après leur isolement, à température élevée et sous pression élevée, en présence d'un catalyseur d'hydrogénation de manière à obtenir les pyrrolidine-2-ones I.
